(19) Europäisches Patentamt
European Patent Office
Office européen des brevets

(11) Veröffentlichungsnummer : **0 039 894**
**B1**

(12) # EUROPÄISCHE PATENTSCHRIFT

(45) Veröffentlichungstag der Patentschrift : **25.07.84**

(51) Int. Cl.[3] : **C 07 J 13/00**, C 12 P 33/08, C 12 P 33/10

(21) Anmeldenummer : **81103423.0**

(22) Anmeldetag : **06.05.81**

(54) **Neue delta-17(20)-BNC-Verbindungen und Verfahren zu ihrer Herstellung.**

(30) Priorität : **12.05.80 AT 2534/80**

(43) Veröffentlichungstag der Anmeldung : **18.11.81 Patentblatt 81/46**

(45) Bekanntmachung des Hinweises auf die Patenterteilung : **25.07.84 Patentblatt 84/30**

(84) Benannte Vertragsstaaten : **BE CH DE FR LI NL**

(56) Entgegenhaltungen :
**DE-A- 2 839 033**
**US-A- 3 994 933**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Bahn, Michael, Dr.**
**Frans-Hals-Weg 19**
**D-4010 Hilden (DE)**
Erfinder : **Preuss, Wolfgang, Dr.**
**Finkenweg 12**
**D-4019 Monheim (DE)**
Erfinder : **Schmid, Rolf, Dr.**
**Am Nettchesfeld 30**
**D-4000 Düsseldorf 13 (DE)**
Erfinder : **Wagner, Rüdiger, Dr.**
**Haferbuckel 27**
**D-4019 Monheim (DE)**

**EP 0 039 894 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 17-substituierte Steroidverbindungen mit einer Sauerstoff-Funktion in 11-Stellung sowie das Verfahren zu ihrer Herstellung.

Es ist bekannt, die Oxidation der Position 11 in Steroidverbindungen auf mikrobiellem Wege durchzuführen. In der Fachliteratur ist eine Vielzahl solcher Steroidoxidationen beschrieben. Verwiesen wird auf die folgenden Literaturreferate sowie die dort genannten Originalveröffentlichungen :

F. Drawert « Biosynthese von Hydroxy-Verbindungen » ; Houben-Weyl « Methoden der organischen Chemie » (1978) 6/1d, Seiten 378-388

T. H. Stoudt, Adv. Appl. Microbiol. 2 (1960) Seiten 190-195 sowie

Handbuch W. Charney und H. L. Herzog « Microbial Transformations of Steroids » Academic Press (1967) New York, Seite 29.

Beschrieben wird hier mit zahlreichen Verweisungen auf bestimmte Mikroorganismenstämme, insbesondere aus der Klasse der Fungi, die mikrobielle 11-Hydroxylierung verschiedenartiger Steroidverbindungen und die dabei entstehenden Syntheseprodukte.

In der DE-OS 28 39 033 ist u. a. die 11-Hydroxylierung von 3-Oxo-4-pregnen-20-carbonsäure (Δ4-BNC) und 3-Oxo-1,4-pregna-dien-20-carbonsäure (Δ1,4-BNC) auf mikrobiologischem Wege beschrieben. Die genannten Ausgangsverbindungen werden hierzu unter Belüftung in wäßrigen Nährlösungen mit Mikroorganismenkulturen behandelt, die zur 11-Hydroxylierung befähigt sind. BNC-Verbindungen dieser Art zeichnen sich dadurch aus, daß der in 17-Stellung vorliegende α-Propionsäurerest mit einer gesättigten C-Bindung an das Steroidringgerüst gebunden ist.

Die Erfindung geht von der Aufgabe aus, strukturähnliche BNC-Verbindungen, die jedoch eine zusätzliche Doppelbindung in 17(20)-Stellung aufweisen, in Position 11 mit Sauerstoff zu funktionalisieren. Diese neuen Verbindungen der Erfindung sind aufgrund ihrer zusätzlichen Doppelbindung in 17(20)-Stellung insbesondere als Zwischenprodukte für die Herstellung von pharmakologisch aktiven Steroidverbindungen geeignet. Die mehrfachen reaktiven Zentren dieser Verbindungen und insbesondere gerade auch die Doppelbindung in 17(20)-Stellung erleichtern ihre Umwandlung in bekannte pharmakologisch wirksame Komponenten der Steroidgruppe.

Gegenstand der Erfindung sind dementsprechend in einer ersten Ausführungsform neue Δ4,17(20)- und Δ1,4,17(20)-BNC-Verbindungen der allgemeinen Formel I.

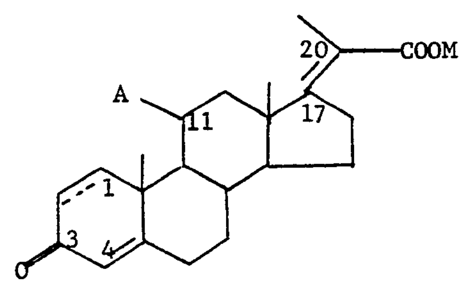

(I)

In dieser Formel I bedeuten A eine Hydroxylgruppe — und zwar sowohl eine α-Hydroxylgruppe als auch eine β-Hydroxylgruppe —, oder A bildet gemeinsam mit dem durch A substituierten C-Atom in 11-Stellung des Steroidringgerüstes eine Carbonylgruppe. M kann verschiedene Bedeutungen haben, insbesondere bedeutet M Wasserstoff, in diesem Fall handelt es sich bei den Verbindungen der allgemeinen Formel I um die freien Carbonsäuren. M kann weiterhin eine salzbildende Gruppe, d. h. ein Kation sein. Als Kationen kommen sowohl Metallkationen, insbesondere Alkalimetall und/oder Erdalkalimetallionen als auch Ammonium oder organische Kationen in Betracht. M kann weiterhin einen Kohlenwasserstoffrest darstellen, der vorzugsweise nicht mehr als 20 C-Atome und insbesondere nicht mehr als 10 C-Atome besitzt. Besondere Bedeutung haben einfache Alkylreste, beispielsweise $C_1$ bis $C_5$, wobei dem Methylrest besondere Bedeutung zukommen kann.

Gegenstand der Erfindung ist in einer weiteren Ausführungsform das Verfahren zur Herstellung neuer Δ4,17(20)- und/oder Δ1,4,17(20)-BNC-Verbindungen der allgemeinen Formel I.

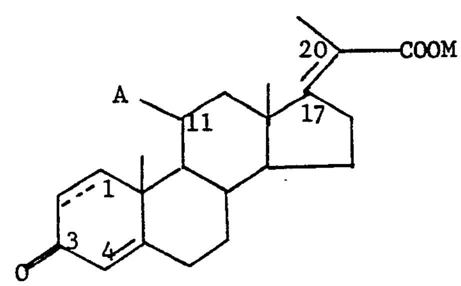

(I)

in der A Hydroxyl oder gemeinsam mit dem durch A substituierten C-Atom eine Carbonylgruppe und M Wasserstoff, einen vorzugsweise niederen Alkylrest, insbesondere den Methylrest, oder eine salzbildende Gruppe bedeuten. Das erfindungsgemäße Verfahren ist dadurch gekennzeichnet, daß man die strukturanalogen, in 11-Stellung jedoch keinen Sauerstoff aufweisenden BNC-Verbindungen in an sich bekannter Weise mittels zur 11-Hydroxylierung befähigter Mikroorganismen in einem wäßrigen Nährmedium unter aeroben Bedingungen in der 11-Position hydroxyliert und gewünschtenfalls das angefallene Produkt der chemischen Transformation zu den Endprodukten der allgemeinen Formel (I) unterwirft.

Die Ausgangsmaterialien für das Verfahren der vorliegenden Erfindung haben die folgende Struktur

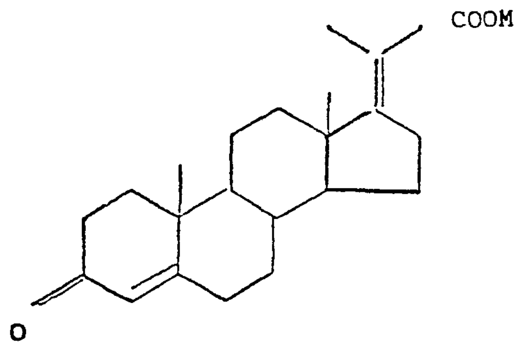

Δ4,17(20)-BNC

und

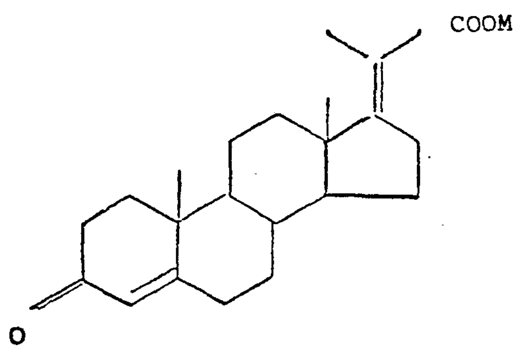

Δ1,4,17(20)-BNC

Die US-PS 3 994 933 beschreibt die Herstellung von 3-Oxo-pregna-4-17(20)-dien-20-carbonsäure (Δ4,17(20)-BNC), ihren niederen Alkylestern und pharmakologisch verträglichen Salzen. Die Säure wird durch mikrobiellen Seitenkettenabbau von 17-C-Steroidverbindungen gewonnen.

In den Europäischen Patentanmeldungen No. 81 100 146.0 (EP-A-33439) und No. 81 100 145.2 (EP-A-34248) wird die Gewinnung von 20-Carboxy-pregna-1,4,17(20)-trien-3-on (Δ1,4,17(20)-BNC) durch mikrobiellen Seitenkettenabbau an 17-C-Seitenkettensteroidsubstraten beschrieben. Geschildert ist dort weiterhin die Umwandlung der 20-Carbonsäuregruppe in den entsprechenden Ester bzw. das Carbonsäurechlorid.

Diese Ausgangsverbindungen werden erfindungsgemäß durch mikrobielle Oxidation zu Verbindungen der allgemeinen Formel I modifiziert.

Für eine hohe pharmakologische Wirksamkeit ist im allgemeinen die Konfiguration 11 β-Hydroxyl oder 11-Oxo erwünscht. Um in Position 11 β-hydroxylierte Steroide zu erhalten, werden entweder Mikroorganismenstämme verwendet, die eine derartige Hydroxylgruppe stereoselektiv einführen, oder es werden andere Mikroorganismen verwendet, die überwiegend oder ausschließlich stereoselektiv in 11 α-Position hydroxylieren. In diesem Fall wird zur Gewinnung der 11 β-hydroxylierten Steroide zunächst chemisch zum 11-Keton oxydiert, und anschließend mit einem geeigneten Reduktionsmittel reduziert, wobei stereoselektiv die 11 β-Hydroxyverbindung gebildet werden kann. Zur einschlägigen Literatur dieser anschließenden chemischen Umwandlung wird beispielsweise auf

L. F. Fieser, M. Fieser « Steroide » Verlag Chemie (Weinheim 1961) Seiten 737 ff. sowie die dort zitierte Originalliteraturstelle J. Am. Chem. Soc. 77, 4436 (1955) verwiesen.

Auch die erfindungsgemäß eingesetzten BNC-Ausgangsverbindungen mit der zusätzlichen olefinischen Doppelbindung in 17(20)-Stellung können weitgehend selektiv in 11-Stellung oxidiert werden. Die bestimmte Struktur der Oxidationsprodukte wird durch die ausgewählten Mikroorganismen bestimmt. Es können demgemäß 11 α-Hydroxyverbindungen, 11 β-Hydroxyverbindungen oder Gemische dieser Komponenten erhalten werden. Auch die 11-Oxoverbindungen können unmittelbares Oxidationsprodukt sein, möglich ist aber auch ihre Herstellung in getrennter Verfahrensstufe durch chemische Oxidation aus zuvor gewonnenen 11 α- und/oder β-Hydroxyverbindungen.

Die 11-Oxidierung mit Mikroorganismen insbesondere aus der Klasse Fungi kann unter literaturbekannten Züchtungsbedingungen erfolgen, verwiesen wird in diesem Zusammenhang auf Rehm « Industrielle Mikrobiologie » Springer (1967), Seiten 518-538 sowie die dort referierte Originalliteratur.

Die Umwandlung der in 11-Stellung unsubstituierten Ausgangsverbindungen erfolgt durch Züchtung, beispielsweise der literaturbekannten für diese Transformation geeigneten Mikroorganismen in einem wäßrigen Nährmedium üblicher Zusammensetzung unter aeroben Bedingungen in Gegenwart der zu transformierenden BNC-Ausgangsverbindungen.

Als BNC-Ausgangsmaterial können sowohl die entsprechenden freien Säuren — bzw. ihre in der wäßrigen Nährlösung löslichen bzw. teillöslichen Salze — aber auch unmittelbar ein Ester, insbesondere der Methylester der freien Säure eingesetzt werden. Bevorzugt wird allerdings mit der freien Säure bzw. ihren im wäßrigen Nährmedium wenigstens anteilsweise löslichen Salzen gearbeitet.

Die Durchführung der mikrobiellen Transformation erfolgt in an sich bekannter Weise, wie es in der zitierten Literatur im einzelnen beschrieben ist. So kann also beispielsweise die als Einsatzmaterial gewählte Steroidverbindung der Kultur während der Inkubationsperiode zugesetzt werden oder sie kann dem Nährmedium vor der Inoculierung der ausgewählten Mikroorganismen beigegeben werden. Es kann eine Steroidverbindung oder auch eine Mischung von mehreren Steroidverbindungen eingesetzt werden. Vorzugsweise werden die Steroidverbindungen in der Kultur in Mengen von etwa 0,1-100 g/l verwendet. Die optimale Konzentration der zu transformierenden Ausgangsverbindung in der Züchtungsstufe ist im allgemeinen stammabhängig und kann durch einfache Vorversuche jeweils ermittelt werden. Im allgemeinen liegt die Konzentration der Steroidausgangsverbindung in Medium vorzugsweise nicht über 50 g/l und häufig nicht über 25 g/l, jedoch kann es bevorzugt sein, mit Mengen über 1 g/l zu arbeiten.

Es kann dabei weiterhin bevorzugt sein, das der Oxidationsreaktion zu unterwerfende Substrat nicht auf einmal dem Reaktionsmedium zuzusetzen, sondern diese Zugabe nach und nach im Verlauf der Reaktion vorzunehmen. Vorzugsweise wird das Ausgangssubstrat in dieser Ausführungsform in wesentlichen kontinuierlich dem Reaktionsgemisch im Verlauf der oxydativen Umwandlung zugegeben. Auf diese Weise kann haüfig die Ausbeute der gewünschten Produkte erhöht werden.

Die Kultur wird in einem Nährmedium gezüchtet, das eine konventionelle metabolisierbare Kohlenstoffquelle sowie die üblicherweise von diesen Mikroorganismen benötigten Nähr- und Wuchsstoffe enthält. Besonders günstig für das Wachstum der Mikroorganismen sind z. B. Glukose, Fruktose, Saccharose, Glycerin, Stärke, Dextrin und zuckerhaltige Abfallstoffe. Geeignete Stickstoffquellen sind Ammoniumsalze, Nitrate, Pepton, Maisquellwasser, Sojamehl, Schlempe und Fischmehl. Weiterhin können Fermentationsbeschleuniger wie Hefeextrakt und Vitamine zugesetzt werden. Das Nährmedium enthält darüberhinaus zweckmäßigerweise anorganische Salze, wie Natrium-, Kalium- oder Ammoniumphosphate.

Die Emulgierung des Ausgangsmaterials im Nährmedium erfolgt vorzugsweise mittels bekannter Emulgatoren, z. B. mittels Fettsäure-Sorbitanester oder deren Ethylenoxidaddukten, Polyoxyethylenmonolaurylether oder Fettsäureethanolamide.

Das verwendete Kulturmedium wird vor Beginn der Bakterienanzucht zweckmäßigerweise durch Erhitzen sterilisiert. Nach dem Abkühlen und Beimpfen des Kulturmediums mit einer geeigneten Vorkultur des transformierenden Mikroorganismenstammes wird zwischen 25-55 °C inkubiert, vorzugsweise bei 27-30 °C. Der pH-Wert der Nährlösung liegt zwischen pH 4-8,5. Die Kultur wird durch Schütteln, Rühren oder Gaseinleiten mit Sauerstoff versorgt und bis zum gewünschten Ausmaß der Oxidationsreaktion inkubiert. Die Oxidationsreaktion fordert je nach Substrat, Konzentration und den anderen Fermentationsbedingungen in der Regel 24-160 Stunden.

Das auf diese Weise gewonnene Oxidationsprodukt, das sich üblicherweise in der Fermentationsbrühe anreichert, kann dann in an sich bekannter Weise aus dem Reaktionsgemisch gewonnen werden. So können die oxidierten BNC-Verbindungen durch Extraktion mit organischen Lösungsmitteln gewünschtenfalls unter Mitverwendung von Austauschersäulen aus dem Reaktionsprodukt isoliert werden. Einzelheiten hierzu finden sich beispielsweise in der am 31.10.1979 veröffentlichten Europäischen Patentanmeldung 004913 — dort geschildert anhand der Isolierung der gebildeten BNC-Verbindungen.

Besonders einfach gelingt auch im Rahmen der vorliegenden Erfindung die Isolierung der hydroxylierten BNC-Verbindungen aus der Fermenterflüssigkeit durch Ausfällung im sauren Bereich.

Die isolierten BNC-Verbindungen mit einer Sauerstoffunktion in 11-Stellung können durch Umkristallisation gereinigt werden.

Besonders wichtige Verbindungen der allgemeinen Formel I sind die 11 β-Hydroxy-Δ-1,4,17(20)-BNC, ihre Salze und Ester. Eine weitere besondere wichtige Verbindung im Rahmen der Erfindung ist die 11 Oxo-Δ1,4,17(20)-BNC und die zuvor erwähnten entsprechenden Derivate dieser Säure.

Die erfindungsgemäßen Verbindungen können insbesondere Ausgangsmaterialien für eine Umwandlung des Substituenten in 17-Stellung und/oder für eine Addition an der 17(20)-Doppelbindung sein. Sie sind damit wertvolle Zwischenprodukte, beispielsweise bei der Herstellung von pharmakologisch aktiven Verbindungen des Progesteron-Typs.

Für die Durchführung des erfindungsgemäßen Verfahrens geeignete Mikroorganismenkulturen sind beispielsweise die zur 11 β-hydroxylierung befähigten Pilzkulturen der Gattungen Curvularia, Cunninghamella und die zur 11 α-Hydroxylierung befähigten Pilzkulturen der Gattungen Aspergillus, Sclerotium, Glomerella, Trichothecium, Absidia oder Rhizopus wie zum Beispiel : Curvularia lunata (NRRL 2380), Cunninghamella blakesleana (ATCC 8688b), Cunninghamella bertholletiae (NRRL 1378), Cunninghamella verticillata (ATCC 8983), Cunninghamella elegans (ATCC 9245), Aspergillus ochraceus (NRRL 405),

Aspergillus niger (NRRL 3228), Sclerotium hydrophilum (IFO 5293), Glomerella cingulata (ATCC 10 534), Trichothecium roseum (ATCC 8685), Absidia orchidis (ATCC 6811) und Rhizopus stolonifer (ATCC 6227b).

Unter den für diese Mikroorganismen üblicherweise verwendeten Kulturbedingungen werden in einem geeigneten Nährmedium unter Belüften Submerskulturen angezüchtet. Dann setzt man den Kulturen das Substrat in der eingangs dargestellten Weise zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Steroidumwandlungen allgemein erfoderlich ist, im Einzelfall durch Vorversuche, wie sie dem Fachmann geläufig sind, ermittelt werden.

## Beispiel 1

11 β-Hydroxy-Δ1,4,17-BNC

Der Stamm Cunninghamella blakesleeana ATCC 8688a wurde in 2 l-Erlenmeyerkolben mit 600 ml Nährlösung folgender Zusammensetzung aerob gezüchtet :

0,15 Gew.-% $KH_2PO_4$
0,20 Gew.-% $NaNO_3$
0,30 Gew.-% Pepton
0,50 Gew.-% Hefeextrakt
3,00 Gew.-% Glucose
pH 5,8

Die Kultur wurde auf der Schüttelmaschine (Schüttelfrequenz 140 UpM) bei 30 °C für 48 Stunden vorgezüchtet, anschließend 0,1 Gew.-% « Tween 80 » (Polyoxyethylensorbitanmonooleat) und 0,2 Gew.-% Δ1,4-BNC mit einem Gehalt an 20 % Δ1,4,17-BNC zugegeben und für weitere 72 Stunden bebrütet. Als Transformationsprodukte wurden erhalten (% der eingesetzten Δ1,4,17-BNC) :

55 % 11 β-Hydroxy-Δ1,4,17-BNC

Die Gewinnung der Reinprodukte erfolgte dabei in diesem und den folgenden Beispielen in der folgenden Weise :

Nach Abbruch der Biotransformation wurden die Zellen mittels Zentrifugation (5 min mit 10 000 g) abgetrennt und das Zentrifugat (1 l) mit 2 × 1 l Dichlormethan im Scheidetrichter extrahiert. Die abgetrennte Zellmasse wurde ebenfalls mit Methylenchlorid oder THF extrahiert. Die vereinigten organischen Extrakte brachte man mit Hilfe eines Rotationsverdampfers im Vakuum zur Trockne.

Der sirupöse Rückstand (2 g) wurde in 150 ml eines Gemisches aus Methanol/Dichlormethan (7 : 3) gelöst und über 25 ml eines Anionenaustauschers Dowex 1 × 2 in folgender Weise gereinigt :

1. Entfernung unerwünschter Medienbestandteile, z. B. Emulgatoren, durch Percolation unter Verwendung weiterer 4 × 25 ml eines Lösungsmittelgemisches aus Methanol/Dichlormethan (7 : 3).

Die gereinigten Percolate werden verworfen.

2. Elution der hydroxylierten BNC-Derivate mit 250 ml eines Gemisches aus 12,3 g HCl conz., 30 ml Dichlormethan, 4,8 g Wasser, mit Methan auf 250 ml aufgefüllt.

Man engte des Eluat auf 50 ml ein und fällte die hydroxylierten BNC-Derivate durch Zugabe von 150 ml destillierten Wassers aus.

Die wäßrige Lösung wurde mit 2 × 100 ml Dichlormethan extrahiert und die vereinigten organischen Extrakte im Rotationsverdampfer in Vakuum zur Trockne gebracht.

Zur weiteren Aufarbeitung wurden die Methylester der hydroxylierten BNC-Derivate durch Umsetzung von 2 g des Rückstandes mit N-[(Tolylsulfonyl-(4)]-N-methyl-nitrosamid nach Maßgabe des Datenblatts Nr. 7/230/4.5/266 der Fa. Merck/Darmstadt hergestellt.

Nach Entfernung des Lösungsmittels wurde der Rückstand in Dichlormethan aufgenommen (20 mg/ml) und der präparativen Liquid-Chromatographie unter folgenden Bedingungen unterworfen :

a) Säule : Dupont Fertigsäule, Part-Nr. 899000001, Füllung Kieselgel, mittlere Teilchengröße 7 u, Säulenlänge 250 mm, äußerer ∅ 25,4 mm

b) Elutionsmittel : Isooctan/Isopropanol (95 : 5)

c) Bedingungen : 50 ml/min Durchfluß, 70 bar, ca. 313 K

d) Detektion : UV-Detektor, 254 Nanometer

Die so aufgetrennte Fraktion wurde dann wie im folgenden beschrieben nach verschiedenen Analysenverfahren charakterisiert.

Beispiel 2

11-Oxo-Δ1,4,17-BNC

10 mg 11 ß-Hydroxy-Δ1,4,17-BNC werden in 1 ml abs. $CH_2Cl_2$ gelöst und rasch zu einer Aufschlämmung von 10 mg Pyridinium-chlorochromat in 1 ml abs. $CH_2Cl_2$ gegeben. Die Suspension wird 1 h bei Raumtemperatur gerührt und dann über eine kurze Kieselgelsäule filtriert (Elutionsmittel : $CH_2Cl_2/CH_3OH$ 4 : 1). Nach Einengen des Eluats verbleiben 8 mg kristallinen Rückstands, der nach dünnschichtchromatographischer Analyse rein ist.

Nach Angaben von Beispiel 1 wurde aus dieser Verbindung der Methylester hergestellt.

Elementarzusammensetzung und Strukturdaten der beiden Verbindungen gehen aus Tabelle 1 hervor.

(Siehe Tabelle 1, Seite 7 f.)

Tabelle 1

Charakterisierung der Produkte

| | Elementarzusammensetzung % C | Elementarzusammensetzung % H | 1 H-NMR-Spektrum[1] chem. Verschiebung | 1 H-NMR-Spektrum[1] Zuordnung | 1 H-NMR-Spektrum[1] Multiplizität | IR-Spektrum[2] Estergruppe | IR-Spektrum[2] Ketogruppe C11 | IR-Spektrum[2] OH | Massenspektrum[3] $M^+$ | Massenspektrum[3] m/e[4] |
|---|---|---|---|---|---|---|---|---|---|---|
| 11ß-Hydroxy-1,4,17-BNC-methylester | ber. 74,56 | 8,16 | 1,21 | 18-$CH_3$ | s | 1720 | - | 3560 | 370 | 121,293 |
| | gef. 74,6/74,6 | 8,36/8,38 | 1,46 | 19-$CH_3$ | s | | | | | |
| | | | 1,94 | 21-$CH_3$ | t (J=2.0 $H_z$) | | | | | |
| | | | 3,68 | 0-$CH_3$ | s | | | | | |
| | | | 4,43 | 11-CH | m | | | | | |
| | | | 6,02<br>6,19/6,21<br>6,31/6,34<br>7,18/7,31 | ABC-System von 1-CH, 2-CH und 4-CH | | | | | | |
| 11-Oxo-1,4,17-BNC-methylester | ber. 74,98 | 7,66 | | | | 1736 | 1707 | | 368 | |
| | gef. 75,1/75,0 | 7,84/7,86 | | | | | | | | |

1) 80 MHz Lösung in $CDCl_3$; Angabe der chemischen Verschiebung in ppm in der δ-Skala, bezogen auf Tetramethylsilan als internem Standard.

2) Aufgenommen in KBr; Angaben in Wellenzahl ($cm^{-1}$).

3) Direktverdampfung.

4) prominente Peaks.

**Ansprüche**

1. Δ4,17(20)- und Δ1,4,17(20)-BNC-Verbindungen der allgemeinen Formel I

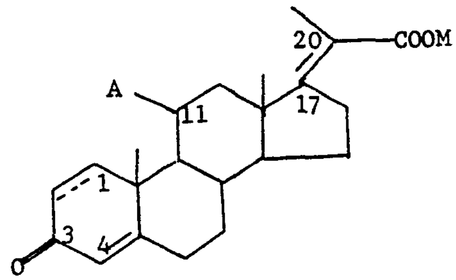

(I)

in der A α-Hydroxyl, β-Hydroxyl oder gemeinsam mit dem durch A substituierten C-Atom eine Carbonylgruppe und M Wasserstoff, eine salzbildende Gruppe oder einen Kohlenwasserstoffrest mit nicht mehr als 20 C-Atomen bedeuten.

2. 11 β-Hydroxy-Δ1,4,17(20)-BNC.

3. 11-Oxo-Δ1,4,17(20)-BNC.

4. Verfahren zur Herstellung neuer Δ4,17(20)-und/oder Δ1,4,17(20)-BNC-Verbindungen der allgemeinen Formel I

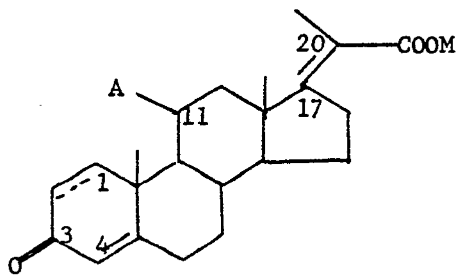

(I)

in der A eine Hydroxylgruppe oder gemeinsam mit dem durch A substituierten C-Atom eine Carbonylgruppe und M Wasserstoff, einen niederen Alkylrest oder eine salzbildende Gruppe bedeuten, dadurch gekennzeichnet, daß man die strukturanalogen, in 11-Stellung jedoch keinen Sauerstoff aufweisenden BNC-Verbindungen in an sich bekannter Weise mittels zur 11-Hydroxylierung befähigter Mikroorganismen in einem wäßrigen Nährmedium unter aeroben Bedingungen in der 11-Position hydroxyliert und gewünschtenfalls das angefallene Produkt der chemischen Transformation zu den Endprodukten der allgemeinen Formel I unterwirft.

5. Verfahren nach Anspruch 4, dadurch gekennzeichnet, daß man die 11-hydroxylierten BNC-Verbindungen der allgemeinen Formel I zu den 11-Oxo-Verbindungen oxidiert.

6. Verfahren nach Ansprüchen 4 oder 5, dadurch gekennzeichnet, daß man die freien BNC-Saüren (M = H) bzw. ihre im wäßrigen Nährmedium löslichen Salze der 11-Hydroxylierung unterwirft und gewünschtenfalls anschließend die 20-Carboxylgruppe zur Estergruppe umwandelt.

**Claims**

1. Δ4,17(20)- and Δ1,4,17(20)-BNC-compounds corresponding to the following general formula

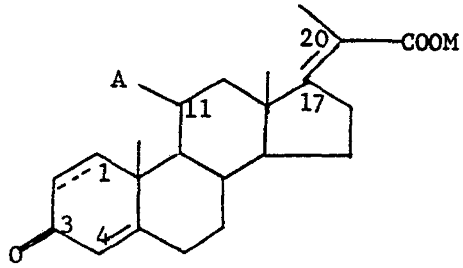

(I)

in which A represents α-hydroxyl, β-hydroxyl or, together with the C-atom substituted by A, a carbonyl group and M represents hydrogen, a salt-forming group or a hydrocarbon radical containing no more than 20 C-atoms.

2. 11 β-Hydroxy-Δ1,4,17(20)-BNC.

3. 11-Oxo-Δ1,4,17(20)-BNC.

4. A process for the production of new Δ4,17(20)- and/or Δ1,4,17(20)-BNC-compounds corresponding to the following general formula

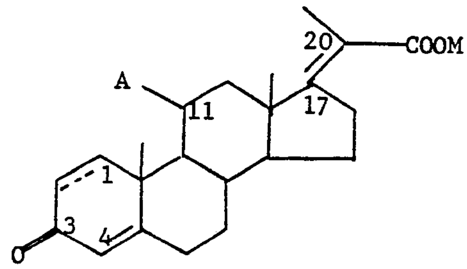

(I)

in which A represents a hydroxyl group or, together with the C-atom substituted by A, a carbonyl group and M represents hydrogen, a lower alkyl radical or a salt-forming group, characterized in that the BNC-compounds of analogous structure, but without any oxygen in the 11-position, are hydroxylated in the 11-position in known manner under aerobic conditions in an aqueous nutrient medium using microorganisms capable of 11-hydroxylation and, if desired, the product formed is subjected to chemical transformation into the end products of general formula I.

5. A process as claimed in Claim 4, characterized in that the 11-hydroxylated BNC-compounds corresponding to general formula I are oxidized to form the 11-oxo-compounds.

6. A process as claimed in Claim 4 or 5, characterized in that the free BNC-acids (M = H) or their salts soluble in the aqueous nutrient medium are subjected to the 11-hydroxylation and, if desired, the 20-carboxyl group is subsequently converted into the ester group.

**Revendications**

1. Composés du type Δ4,17(20)- et Δ1,4,17(20)-BNC, de formule générale

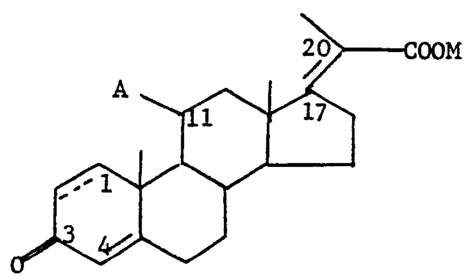

(I)

dans laquelle A représente un groupe α-hydroxyle, β-hydroxyle ou, avec l'atome de carbone portant A comme substituant, un groupe carbonyle, et M représente un atome d'hydrogène, un groupe générateur de sel ou un radical d'hydrocarbure n'ayant pas plus de 20 atomes de carbone.

2. 11 β-hydroxy-Δ1,4,17(20)-BNC.

3. 11-oxo-Δ1,4,17(20)-BNC.

4. Procédé de préparation de nouveaux composés de type Δ4,17(20)- et/ou Δ1,4,17(20)-BNC de formule générale I

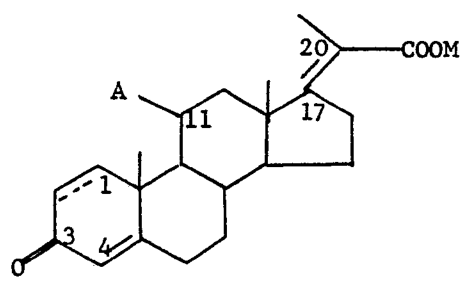

(I)

dans laquelle A représente un groupe hydroxyle ou, avec l'atome de carbone portant A comme substituant, un groupe carbonyle, et M représente de l'hydrogène, un radical alkyle inférieur ou un groupe générateur de sel, procédé caractérisé en ce qu'on hydroxyle en position 11 dans des conditions aérobies, dans un milieu nutritif aqueux, de façon connue en soi, à l'aide de micro-organismes capables d'une hydroxylation en position 11, des composés de type BNC de structure analogue mais ne présentant

cependant pas d'oxygène en position 11 et, éventuellement, on soumet le produit obtenu à une transformation chimique pour obtenir les produits finals de formule générale I.

5. Procédé selon la revendication 4, caractérisé en ce qu'on oxyde les composés de type BNC, hydroxylés en position 11, de formule générale I, pour obtenir les composés de type 11-oxo.

6. Procédé selon les revendications 4 ou 5, caractérisé en ce qu'on soumet les acides libres de type BNC (M = H) ou leurs sels solubles dans le milieu nutritif aqueux à une hydroxylation en position 11 et l'on transforme éventuellement ensuite le groupe 20-carboxyle en un groupe ester.